(19) Europäisches Patentamt
European Patent Office
Office européen des brevets

(11) Publication number: 0 154 409 A2

(12) # EUROPEAN PATENT APPLICATION

(21) Application number: 85300748.2

(22) Date of filing: 05.02.85

(51) Int. Cl.⁴: C 12 Q 1/32

(30) Priority: 06.02.84 JP 20367/84
14.02.84 JP 26559/84

(43) Date of publication of application:
11.09.85 Bulletin 85/37

(84) Designated Contracting States:
DE GB NL

(71) Applicant: UNITIKA LTD.
No. 50, Higashihonmachi 1-chome
Amagasaki-shi Hyogo(JP)

(72) Inventor: Watanabe, Mitsuo
32, Satojiri Uji
Uji-shi Kyoto(JP)

(72) Inventor: Suzuki, Tadao
151-30-3D-1109, Nimaru-cho Mukaijima
Mukaijima, Fushimi-ku, Kyoto-shi, Kyoto(JP)

(72) Inventor: Kageyama, Masao
55, Kageyama Uji
Uji-shi Kyoto(JP)

(74) Representative: Pearce, Anthony Richmond et al,
Marks & Clerk Alpha Tower Suffolk Street
Queensway Birmingham B1 1TT(GB)

(54) **Testing material for detecting alcohols.**

(57) A testing material for detecting alcohols is described, comprising (a) NAD or NADP, (b) alcohol dehydrogenase, (c) (1) thermophilic microorganism-derived diaphorase, or (2) diaphorase and dextran, (d) a dye and (e) a carrier.

This testing material has very good storage stability and can measure alcohol concentrations, in particular ethanol concentrations in saliva, enzymatically with ease. It is useful in detecting and preventing driving while under the influence of liquor.

EP 0 154 409 A2

# TESTING MATERIAL FOR DETECTING ALCOHOLS

## FIELD OF THE INVENTION

This invention relates to a testing material for enzymatically determining alcohol concentrations, particularly ethanol concentrations, in solutions.

## BACKGROUND OF THE INVENTION

It is a frequent practice in the food and chemical industries to measure alcohol concentrations in solutions for the purpose of process control. Among alcohols, determination of ethanol concentrations is a routine test item in the fields of medicine and clinical laboratory testing. It is also an important test item in the area of traffic laws and regulations.

A number of chemical methods in current use for the determination of alcohols in solutions use a volumetric determination or color change of an oxidizing agent resulting from the oxidation of alcohols. These methods each has a serious drawback in that the oxidizing agent can also react with various volatile substances other than alcohols that may be present, and therefore such methods are lacking in specificity.

For that reason, methods for determining alcohols based on gas chromatography or liquid chromatography were developed in the 1960's as physicochemical

methods of specifically determining particular substances only. While such methods give exact determination results and are specific, they have difficulties in many aspects; for instance, much time is required, expensive particular apparatus and training of personnel to render them capable of handling such apparatus are required, and the methods are lacking in generality, instant applicability, and/or practicability.

On the other hand, biochemical methods of analysis using enzymes are generally superior in specificity to chemical and physicochemical methods and are advantageous in that no particular or large-sized apparatus are required. Among such methods hitherto reported, there are a method using alcohol dehydrogenase (R. Bonnichsen et al., Scand. J. Clin. Lab. and Invest., p. 358, 1951) and a method using alcohol oxidase (Frank W. Janssen et al., Biochim. Biophys. Acta, Vol. 151, pp. 330-342, 1968). According to these methods, the enzymatic reaction is carried out in solution and the resulting reduced nicotinamide adenine dinucleotide (hereinafter abbreviated as NADH) is measured at 340 nm or the resulting hydrogen peroxide is reacted with orthodianisidine followed by measurement of the color formed thereby at 436 nm. As a method of determining NADH, the combined use of diaphorase and a dye is also

reported (F. Kaplan et al., Arch. Biochim. Biophys., Vol. 132, p. 91, 1969). Furthermore, there has been a proposal to determine ethanol in body fluids using an enzyme solution containing alcohol dehydrogenase, NAD, diaphorase, and a tetrazolium dye (British Patent 1,351,547). However, each of the foregoing is an optical method, and therefore necessarily require use of an apparatus such as a spectrophotometer. Moreover, such methods cannot be applied to turbid samples such as blood or food samples. Furthermore, for carrying out the enzymatic reaction, a troublesome procedure involving dissolution of various reagents and distribution of the resulting solutions into reaction vessels in amounts specified beforehand is required, and the reagent solutions prepared have poor storage stability. For these and other reasons, the above-mentioned methods are not suited for general use.

To overcome these difficulties, the combined use of an enzymatic method and an electrochemical method for determining alcohols has been desired. Thus, for instance, an enzyme electrode method described in Japanese Patent Publication No. 56019/82 uses alcohol dehydrogenase. This method determines the change of oxidation reduction potential caused by hydrogen formed from ethanol by an enzymatic reaction using an electrical

method, e.g., pH meters, and requires an enzyme-immobilizing membrane and an electric measuring apparatus. The enzyme membrane is poor in durability; a liquid enzyme membrane is stable at 4°C for only 2 days and an immobilized enzyme-carrying membrane is stable for only 2 weeks. Such stability is insufficient for practical use of the method. Moreover, it is also apparent that a relatively large amount of sample, namely an amount sufficient to immerse the electrode therein, will be required. In clinical laboratory testing, for instance, the amount of blood that can be taken is limited. In this and the above respects, this method is also lacking in general usability, generality and practicability.

Accordingly, the art has hitherto searched for a method of determining alcohol concentrations with a small amount of sample in an easy and simple manner at any place using a portable device, but without requiring any particular apparatus or skill. However, until now, a method capable of meeting all of the desired characteristics has not been available.

In a recent publication, Japanese Patent Application (OPI) No. 166098/84 (the term "OPI" as used herein refers to a "published unexamined Japanese patent application") (Canadian Patent Application No. 419,293

filed on January 12, 1983; U.S. Patent Application Serial No. 516,295 filed on July 22, 1983; Canadian Patent Application No. 443,221 filed on January 5, 1984), there has been proposed an alcohol-determining composition in solid form as prepared by lyophilizing the enzyme-containing assay solution as described in the above-cited British Patent 1,351,547, either as it is or together with an enzyme activity inhibitor. This alcohol assay composition is indeed superior in ease of the assay procedure and portability, but has insufficient storage stability. It is necessary for said composition to be stored in a dark-colored bottle in a freezer (-15°C) or a refrigerator under dry conditions, and thus the composition is lacking in practicability.

Japanese Patent Application (OPI) No. 7724/81 (corresponding to West German Patent Publication No. 2,925,009, European Patent Publication No. 21,009) describes a preparation suited for use as a pharmaceutical or diagnostic agent that is obtained by using a lyophilization process involving a small number of steps which is carried out in the presence of dextran, to thereby prevent inactivation of sensitive active ingredients such as medicinal substances in a multistep adjustment and preparation process such as tableting, sugar coating, or filling into capsules. However, said

publication discloses nothing about stabilization of testing materials or determination of alcohols.

SUMMARY OF THE INVENTION

An object of the invention is to provide a testing material for detecting alcohols which can be stored even at ordinary room temperature (about 20°C) and has a long life time, i.e., good storage stability.

Another object of the invention is to provide a testing material for detecting alcohols which can determine alcohol concentrations in a simple and easy manner at any place with a small amount of sample, without requiring any particular apparatus or skill, and which is easily portable, e.g., to a site for food manufacturing process control, diagnosis of acute alcoholism, or traffic control.

As a result of intensive investigations to achieve the above objects, it has now been found that a testing material comprising NAD (Nicotinamide Adenine Dinucleotide) or NADP (i.e., NAD Phosphate), alcohol dehydrogenase, thermophilic microorganism-derived diaphorase, a dye, and a carrier, or a testing material comprising NAD or NADP, alcohol dehydrogenase, diaphorase, dextran, a dye, and a carrier, when exposed to alcohols in sample solutions of very small size, develops a color or is decolorized depending on the alcohol content in

the sample solutions, whereby the alcohol concentrations can be determined. Surprisingly, it was also found that the life time (stability) of such testing material under room temperature storage conditions is prolonged to not less than one month, whereby the above objects can be accomplished. These findings have now led to completion of the present invention.

Thus, the invention provides a testing material for detecting alcohols which comprises (a) NAD or NADP, (b) alcohol dehydrogenase, (c) thermophilic microorganism-derived diaphorase, (d) a dye and (e) a carrier, and a testing material for detecting alcohols which comprises (a) NAD or NADP, (b) alcohol dehydrogenase, (c) diaphorase and dextran, (d) a dye, and (e) a carrier.

The alcohol-detecting testing material according to the invention is not only very easily transportable but also enables judgment by the eye of the results of the enzymatic reaction, due to the coexistence of diaphorase and a dye. Therefore, alcohol concentrations can be detected at virtually any site in a simple and easy manner and in a short period of time, without need for a spectrophotometer or any particular electrochemical apparatus. The testing material, when dried, exhibits good storability. When stored at room temperature (37°C), it can remain usable for more than 1 month. This is a

marked improvement in storage stability as compared with a dried testing material of the same sort but lacking in thermophilic microorganism-derived diaphorase or in dextran as a stabilizing agent, which, when stored at 37°C, exhibits a life time as short as 3 days. Moreover, the testing material according to the invention is very high in practicability and is very useful in emergency situations (e.g., diagnosis of acute alcoholism) and in enforcing traffic regulations, so that the benefits provided to society through the use of said testing material are very significant.

DETAILED DESCRIPTION OF THE INVENTION

Upon impregnation of the testing material according to the invention with an alcohol-containing solution, an enzymatic reaction involving the alcohol immediately takes place. The reaction involves a first step in which the alcohol and the coexisting NAD or NADP are converted to the corresponding aldehyde and NADH (reduced form of NAD) or NADPH (reduced form of NADP), respectively, through the mediation of alcohol dehydrogenase (see reaction equation 1 below), and a second step in which diaphorase catalyzes the transfer of hydrogen from the resulting NADH or NADPH to the dye, whereby the dye is decolored or produces a color (see reaction equation 2 below). The alcohol content is

determined by immersing the testing material in an alcohol-containing solution and observing, visually, the extent or rate of decoloration or color development.

Reaction Equation 1

$$\text{Alcohol} + \text{NAD(P)} \underset{}{\overset{\text{Alcohol Dehydrogenase}}{\rightleftharpoons}} \text{NAD(P)H} + \text{Aldehyde}$$

Reaction Equation 2

$$\text{NAD(P)H} + \text{Dye (oxidized form)} \overset{\text{Diaphorase}}{\rightleftharpoons} \text{NAD(P)} + \text{Dye (reduced form)}$$

Addition of aldehyde dehydrogenase to the above composition, so that the carrier can contain the same, can result in increased detection sensitivity and significantly shortened assay time. When this aldehyde dehydrogenase-containing testing material is impregnated with an alcohol-containing solution, the enzymatic reaction involving the alcohol starts immediately. The reaction includes the step of alcohol dehydrogenase-catalyzed conversion of the alcohol to the corresponding aldehyde and of the NAD or NADP to NADH or NADPH (according to reaction equation 1 above), the step of further conversion of the aldehyde formed in the first step to

the corresponding acid in the presence of NAD or NADP with simultaneous formation of NADH or NADPH (see reaction equation 3 below), and the step of diaphorase-catalyzed hydrogen transfer from the NADH or NADPH formed in the preceding step to the dye, which leads to decoloration of the dye or color development (according to reaction equation 2 above).

Reaction Equation 3

$$\text{Aldehyde} + \text{NAD(P)} \xrightleftharpoons{\text{Aldehyde Dehydrogenase}} \text{Acid} + \text{NAD(P)H}$$

Among the enzymes to be used in accordance with the invention, diaphorase is an enzyme classified as NADH: lipoamide oxidoreductase EC 1.6.4.3 or NADH: dye oxidoreductase EC 1.6.99 (formal scientific name and enzyme classification number). Any species of diaphorase which is capable of reducing the dye used may be used provided that it is a thermophilic microorganism-derived one. Where other diaphorase species, such as swine heart-derived diaphorase and mesophile-derived diaphorase (commercially available from Boehringer Mannheim-Yamanouchi and Toyobo), are used, the combined use of dextran is necessary.

The testing material in which thermophile-derived diaphorase and dextran are used is particularly preferred since it has improved storage stability.

The expression "thermophilic microorganism (or thermophile)" means a microorganism optimally growing at a temperature of 40 to 80°C. Examples of such microorganism include microorganisms belonging to the genus Bacillus, such as Bacillus caldoluticus and Bacillus stearothermophilus (e.g., FERM P-7275) (FERM: a deposition number at Fermentation Research Institute, Agency of Industrial Science and Technology, Ministry of International Trade and Industry, Japan), microorganisms belonging to the genus Corynascus, such as Corynascus thermophilus (e.g., ATCC 22066) (ATCC: a deposition number at American Type Culture Collection), microorganisms belonging to the genus Streptomyces, such as Streptomyces thermonitrificans (e.g., ATCC 233845) and Streptomyces thermophilus (e.g., ATCC 19282), microorganisms belonging to the genus Talaromyces, such as Talaromyces emersonii (e.g., ATCC 28080) and Talaromyces thermophilus (e.g., ATCC 20186), microorganisms belonging to the genus Thermoascus, such as Thermoascus aurantiacus (e.g., ATCC 26904), microorganisms belonging to the genus Thermus, such as Thermus aquaticus T351 and Thermus thermophilus (e.g., ATCC 27634), microorganisms belonging to the genus Thielavia, such as Thielavia albomyces (e.g., ATCC 28084), and microorganisms belonging to the genus Humicola, such as Humicola brevis (e.g., ATCC 28402) and Humicola brevispora (e.g., ATCC 28403).

The use of thermophile-derived diaphorase, among others, is preferred. Bacillus stearothermophilus-derived diaphorase, for instance, can be obtained by following the procedure described in Biochem. J., Vol. 191, pp. 457-465, 1980, and is commercially available from Unitika.

Thermus aquaticus-derived diaphorase can be obtained, for instance, by the procedure described in Biochem. J., Vol. 209, pp. 427-433, 1983.

The above-mentioned thermophilic microorganisms also include thermophile-derived enzyme producing mesophiles created by the cell fusion, recombinant DNA, or the like technique.

The above-mentioned alcohol dehydrogenase is an enzyme generally calssified as Alcohol: NAD oxidoreductase EC 1.1.1.1 or Alcohol: NAD(P) oxidoreductase EC 1.1.1.2 (formal scientific name and enzyme classification number), and any species thereof which is capable of dehydrogenating the alcohol to be assayed may be used. Thus, for instance, for assaying ethanol, an equine liver-derived alcohol dehydrogenase and a microorganism-derived alcohol dehydrogenase can equally be used, and such are commercially available on a worldwide basis from Boehringer Mannheim-Yamanouchi and Oriental Yeast. When thermophile-derived alcohol dehydrogenase is used,

it may be purified by a conventional method. For the purification of alcohol dehydrogenase from Bacillus stearothermophilus, for instance, the procedure described by A. Atkinson in Biochem. J., Vol. 127 (3), pp. 63-64, 1972, or by John Bridgen in FEBS Lett., Vol. 33 (1), pp. 1-3, 1973, may be used.

The above-mentioned aldehyde dehydrogenase is an enzyme generally calssified as Aldehyde: NAD oxidoreductase EC 1.2.1.3 or Aldehyde: NADP oxidoreductase EC 1.2.1.4 or Aldehyde: NAD(P) oxidoreductase EC 1.2.1.5 (formal scientific name and enzyme classification number), and any species thereof which is capable of dehydrogenating the aldehyde corresponding to the alcohol to be assayed may be used. For assaying ethanol, for instance, such aldehyde dehydrogenase is commercially available from several companies (e.g., Boehringer Mannheim-Yamanouchi, Sigma, Oriental Yeast).

Regarding NAD, NADP and dextran, reagent grades thereof commercially available from several companies (e.g., Boehringer Mannheim-Yamanouchi, Oriental Yeast, Sigma, Seikagaku Kogyo) may be used. Preferably, dextran has a molecular weight of not less than 60,000. The dye may be any one capable of producing color (chromogen) or being decolored under the action of diaphorase, such as a ferricyanide compound or a so-

called formazan dye. Potassium ferricyanide is an example of the former and iodonitrotetrazolium salt is an example of the latter. Other usable dyes include, but are not limited to, methylene blue, 2,6-dichlorophenol, indophenol, indigo carmine and amaranth. Among them, those dyes the safety of which has been confirmed, namely, indigo carmine, amaranth and methylene blue, each approved as a food additive or in use as a medicinal substance, are preferably used. A testing material for detecting alcohols in which such safety-confirmed dye is used is most appropriate for determining alcohol concentrations in saliva and very advantageous in ethanol assays in enforcing traffic regulations.

In making the testing material according to the invention, the above-described dyes may be used either singly or in admixture.

The carrier for use in the testing material according to the invention may be any carrier material which is insoluble or sparingly soluble in water at ordinary temperature (solubility at 25°C: not more than 1.0%). Thus, for example, naturally occurring low molecular to high molecular materials, typically gelatin, agar, cellulose, paper, chitin and glucan, and semisynthetic or synthetic macromolecules such as rayon, vinylon, polyesters, certain species of polyvinyl

alcohol, nylon and acrylic polymers are all usable. The size, thickness and shape of the testing material are optional.

In making the testing material according to the invention, the reagent concentrations may be as follows: In manufacturing testing material for detecting alcohols in a concentration of 1 mol/liter, the dye concentration generally is from 0.01 to 10 mols/liter, preferably is from 0.1 to 2.0 mols/liter, and most preferably is from 0.2 to 1.0 mol/liter, and the alcohol dehydrogenase, aldehyde dehydrogenase and diaphorase concentrations are each from $10^2$ to $10^{11}$ units/liter, preferably from $10^4$ to $10^9$ units/liter, and most preferably from $10^6$ to $10^8$ units/liter. The NAD or NADP concentration generally is from 0.001 to 2.0 mols/liter, preferably is from 0.01 to 1.5 mols/liter, and most preferably is from 0.1 to 1.0 mol/liter. Dextran can be used in an amount of from 5 to 15% by weight, preferably from 8 to 12% by weight, and most preferably is about 10% by weight. Any buffer capable of dissolving these reagents may be used. The buffer generally has a pH of from 4 to 12, preferably from 6 to 11, and most preferably is from 7.0 to 9.5, and is used at a concentration generally from 0.01 to 2 mols/liter, preferably from 0.1 to 1.0 mol/liter, and most preferably from 0.2 to

0.8 mol/liter. An example of such buffer is tris-hydrochloride buffer.

When the alcohol concentration to be detected is lower than 1 mol/liter, the contents of the respective components may be reduced accordingly. However, such content reduction generally results in prolongation of the time required for the enzymatic reaction to be complete. Therefore, it is generally desirable to reduce the content of the dye alone. Depending on the properties of the enzyme used, NAD and NADP may be used either singly or in combination. The activity of each enzyme to be added is given herein international units, one unit being the enzyme activity that causes one μmol of substrate to react in one minute at 25 to 30°C.

The testing material according to the invention may be produced, for example, by impregnating a carrier insoluble or sparingly soluble in water either partly or wholly in a solution containing the above-described reagents used according to the present invention. After removal from the solution, the testing material may be used either as is or after drying. When a large sized carrier is used, it may be cut, after immersion in the above-mentioned reagent solution, into pieces of material having an adequate size, for example, about 5 mm in width and about 40 mm in length. In drying

the testing material, the drying should recommendably be carried out at low temperature or in the manner of vacuum drying, since the enzymes are thermally unsatble by nature.

In actually determining alcohol concentrations in solutions using a piece of the testing material according to the invention, the piece of the testing material which has reagent concentrations suited for the determination is dipped in the solution to be assayed and then taken out of the solution, and the time required for the dye to fade or develop color at room temperature is measured. The alcohol concentration can also be determined from the change in color tone. In determining alcohol concentrations in saliva, too, the piece of the alcohol-detecting testing material is immersed in saliva, and the time required for fading is measured or the change in color tone is observed by the eye, to thereby determine the alcohol concentration in the saliva.

The following examples illustrate the invention in further detail. The reagents used in the examples were as follows:

1. NAD or NADP: From baker's yeast, biochemical reagent grade purchased from Oriental Yeast;
2. Alcohol dehydrogenase: Oriental Yeast's catalog No. ADH-01;

3. Diaphorase: Mesophile-derived diaphorase (from Clostridium kluyveri), diagnostic reagent grade purchased from Toyobo, and thermophile-derived diaphorase was Unitika's lyophilized product EC 1.6.99.-;

4. Aldehyde dehydrogenase: Boehringer Mannheim-Yamanouchi's catalog No. 171832.

Each unit of alcohol dehydrogenase or aldehyde dehydrogenase is equal to an enzyme quantity required to consume 1 micromol of ethanol or acetaldehyde, respectively, at 25°C, in 1 minute. Each unit of diaphorase corresponds to an enzyme quantity capable of consuming 1 micromol of NAD(P)H at 25°C in the presence of 2,6-dichlorophenolindophenol in 1 minute.

EXAMPLE 1

A solution containing 4 millimols/liter (hereinafter abbreviated as mM) of methylene blue, 340 units/mℓ of alcohol dehydrogenase, 1,000 units/mℓ of mesophile-derived diaphorase, 6 mℓ/liter of Triton X-100, 200 mM of Tris-hydrochloride buffer (pH 9.0) and 10 mM of NAD was prepared, and a piece of filter paper (Toyo Kagaku's filter paper for qualitative determination No. 2; circle, 9 cm in diameter, 0.26 mm in thickness) was impregnated with the solution, dried under vacuum and cut into rectangular pieces, 5 mm in width

and 40 mm in length, to give blue pieces of testing material I.

Following the same procedure as above, but using thermophile-derived diaphorase in lieu of mesophile-derived diaphorase, pieces of testing material II were produced.

On the day of their manufacture, pieces of the testing materials I and II were immersed in 0.5 g/liter aqueous ethanol solution, removed, and allowed to stand at room temperature while being observed by the eye. With both of the pieces of testing material, the blue color of the immersed portion began to fade and turned colorless in 1 minute. The time required for fading was measured while varying the ethanol concentration in the ethanol solution for immersion. The time required for fading became shorter with the increase in ethanol concentration. At very low ethanol concentrations, the fading was incomplete and only a decrease in color tone was noted.

The results obtained are shown in Table 1.

TABLE 1

| Ethanol Concentration | Extent of Fading on Pieces of Testing Materials I and II and Time Required for Fading |
|---|---|
| 0.1 g/liter | Color changed from deep blue to blue in 1 minute, thereafter not changed. |
| 0.4 g/liter | Color changed from deep blue to pale blue in 1 minute, thereafter not changed. |
| 0.5 g/liter | Color changed from deep blue to white in 1 minute. |
| 1.0 g/liter | Color changed from deep blue to white in 40 seconds. |
| 2.0 g/liter | Color changed from deep blue to white in 20 seconds. |

As shown in Table 1, the ethanol concentrations in solutions could be determined with pieces of both the testing materials I and II by observing the change in color tone and measuring the time required for fading.

In a next test, the pieces of testing materials I and II were stored at room temperatures of 4°C or 37°C, respectively, and evaluated for their useful life. The results obtained are shown in Table 2. At 37°C, the testing material I exhibited a life time of only 3 days, whereas the testing material II in which thermophile-derived diaphorase was used did not show any decrease in performance even after 1 month of storage under the same conditions and, after 1 month of storage, gave substantially the same results in determining ethanol concentrations in solution.

TABLE 2

| Storage Conditions | Useful Life: Testing Material I | Useful Life: Testing Material II |
|---|---|---|
| Room Temperature (4°C) | 1 Month | >6 Months |
| Room Temperature (37°C) | 3 Days | >1 Month |

EXAMPLE 2

To evaluate the increase in storability of testing materials as brought about by dextran, the manufacturing procedure of Example 1 was followed, with further addition, to the reagent solution, of 10% by weight of dextran, to provide pieces of testing materials III and IV. The pieces of testing material III carried the composition for testing material I (containing mesophile-derived diaphorase) plus dextran, and the pieces of testing material IV carried the composition for testing material II (containing thermophile-derived diaphorase) plus dextran.

The pieces of testing materials III and IV were evaluated for their useful life at room temperature (37°C) and the results obtained are shown in Table 3. When stored at room temperature (37°C), the useful life of pieces of testing material I (3 days) was extended to 12 days (testing material III) and the useful life of the pieces of testing material II was extended from more than 1 month to more than 4 months (testing material IV), respectively by the addition of dextran.

TABLE 3

| Storage Conditions | Useful Life: Testing Material III | Useful Life: Testing Material IV |
| --- | --- | --- |
| Room Temperature (37°C) | 12 Days | >4 Months |

EXAMPLE 3

The procedure of Example 1 for the manufacture of pieces of testing material I was followed except that *Bacillus stearothermophilus*-derived alcohol dehydrogenase and diaphorase were used as the alcohol dehydrogenase and diaphorase in the composition given for the testing material I in Example 1, to provide pieces of testing material V.

The testing material V was immersed in ethanol solutions differing in ethanol concentration in the same manner as Example 1, and the color changes were followed.

The results obtained were quite comparable to those of Example 1, indicating that thermophile-derived enzymes are also usable in testing materials for detecting alcohols.

The testing material V was superior in storage stability to the testing material II. Namely, it retained its performance characteristics for more than 3 months in a storage test conducted at a temperature of 37°C.

EXAMPLE 4

The increase of the sensitivity of pieces of testing material due to the addition of aldehyde dehydrogenase was evaluated. The pieces of testing material VI was produced in the same manner as in the testing material IV of Example 2 except that aldehyde dehydrogenase was added, in a concentration of 8 units/mℓ, to the composition of the testing material IV in Example 2.

The testing material IV and VI thus obtained were immersed in 0.5 g/liter ethanol solution, then removed and allowed to stand at room temperature, while observing for changes in color tone by the eye. With both materials, the blue color of the immersed portion faded immediately and turned colorless. The time required for the blue color of testing material VI to

fade completely was one third of that for testing material IV, as seen from the data set forth below in Table 4. It is thus evident that the addition of aldehyde dehydrogenase is effective in reducing the time required for the determination.

TABLE 4

| Testing Material | Time Required for Fading Reaction |
|---|---|
| Testing Material IV | 60 seconds |
| Testing Material VI | 20 seconds |

In another test, the concentration of the permeating ethanol solution was varied, whereby the time required for the blue color of testing material VI to fade completely, or the extent of fading, varied. Thus, the time required for fading became shorter with increasing ethanol concentration. In very dilute ethanol concentrations, the fading was not complete, although the color tone became lighter. The results obtained are shown in Table 5.

TABLE 5

| Ethanol Concentration | Extent and Rate of Fading |
|---|---|
| 0.05 g/liter | Color changed from deep blue to blue. |
| 0.10 g/liter | Color changed from deep blue to light blue. |
| 0.20 g/liter | Color changed from deep blue to white in 50 seconds. |
| 0.50 g/liter | Color changed from deep blue to white in 20 seconds. |
| 1.00 g/liter | Color changed from deep blue to white in 10 seconds. |

The data shown in Table 4 and Table 5 confirmed that the testing material VI with aldehyde dehydrogenase further added can assay ethanol more rapidly and with better sensitivity than the aldehyde dehydrogenase-free testing material IV.

EXAMPLE 5

The testing material V obtained in Example 3 was used for determining the alcohol concentration in saliva.

Thus, an adult male weighing 65 kg was given 360 mℓ of "sake" (Japanese rice wine), and, 1 hour after drinking, saliva sampling was conducted. When assayed for ethanol by gas chromatography (using a Shimadzu model

GC-3BT gas chromatograph), the saliva sample gave an ethanol concentration of 0.52 g/liter.

The saliva sample was then allowed to permeate into a piece of testing material V. The deep blue color faded to white in 55 seconds. Referring to the results obtained in Example 1, it was estimated that the saliva sample had an ethanol content of about 0.5 g/liter. This value was in good agreement with the result of the gas chromatography test. It was thus confirmed that ethanol concentrations in saliva can be determined in a simple and easy manner by using the testing material V.

While the invention has been described in detail and with reference to specific embodiments thereof, it will be apparent to one skilled in the art that various changes and modifications can be made therein without departing from the spirit and scope thereof.

CLAIMS:

1. A testing material for detecting alcohols which comprises (a) nicotinamide adenine dinucleotide or nicotinamide adenine dinucleotide phosphate, (b) alcchol dehydrogenase, (c) (1) a thermophilic microorganism-derived diaphorase or (2) a diaphorase and dextran, (d) a dye, and (e) a carrier.

2. A testing material as in claim 1, which further comprises aldehyde dehydrogenase.

3. A testing material as in claim 1, wherein (c) is a thermophilic microorganism-derived diaphorase.

4. A testing material as in claim 2, wherein (c) is a thermophilic microorganism-derived diaphorase.

5. A testing material as in claim 1, wherein the thermophilic microorganism-derived diaphorase is thermophilic bacterium-derived diaphorase.

6. A testing material as in claim 2, wherein the thermophilic microorganism-derived diaphorase is thermophilic bacterium-derived diaphorase.

7. A testing material as in claim 1, wherein (c) is a diaphorase and dextran.

8. A testing material as in claim 7, which further comprises aldehyde dehydrogenase.

9. A testing material as in claim 7, wherein the diaphorase is thermophilic microorganism-derived diaphorase.

10. A testing material as in claim 9, which further comprises aldehyde dehydrogenase.

11. A testing material as in claim 1, wherein the NAD or NADP concentration is from 0.001 to 2.0 mols/liter, the alcohol dehydrogenase and thermophilic microorganism-derived diaphorase concentrations are each from $10^2$ to $10^{11}$ units/liter, and the dye concentration is from 0.01 to 10 mols/liter.

12. A testing material as in claim 7, wherein the NAD or NADP concentration is from 0.001 to 2.0 mols/liter, the alcohol dehydrogenase and thermophilic microorganism-derived diaphorase concentrations are each from $10^2$ to $10^{11}$ units/liter, and the dye concentration is from 0.01 to 10 mols/liter.

13. A testing material as in claim 1, wherein the NAD or NADP concentration is from 0.01 to 1.5 mols/liter, the alcohol dehydrogenase and thermophilic microorganism-derived diaphorase concentrations are each from $10^4$ to $10^9$ units/liter, and the dye concentration is from 0.1 to 2.0 mols/liter.

14. A testing material as in claim 7, wherein the NAD or NADP concentration is from 0.01 to 1.5 mols/liter, the alcohol dehydrogenase and thermophilic microorganism-derived diaphorase concentrations are each from $10^4$ to $10^9$ units/liter, and the dye concentration is from 0.1 to 2.0 mols/liter.

15. A testing material as in claim 1, wherein the NAD or NADP concentration is from 0.1 to 1.0 mol/liter, the alcohol dehydrogenase and thermophilic microorganism-derived diaphorase concentrations are each from $10^6$ to $10^8$ units/liter, and the dye concentration is from 0.2 to 1.0 mol/liter.

16. A testing material as in claim 7, wherein the NAD or NADP concentration is from 0.1 to 1.0 mol/liter, the alcohol dehydrogenase and thermophilic microorganism-derived diaphorase concentrations are each from $10^6$ to $10^8$ units/liter, and the dye concentration is from 0.2 to 1.0 mol/liter.